# EUROPEAN PATENT SPECIFICATION

(11) **EP 0 540 790 B1**
(45) Date of publication and mention of the grant of the patent: **11.09.1996**
(21) Application number: 91310220.8
(22) Date of filing: 05.11.1991
(51) Int. Cl.: C12P 19/26, C12N 9/24, C12N 11/10

(54) **Method of obtaining monosialogangliosides**
Verfahren zur Erlangen von Monosialogangliosiden
Procédé d'obtention de monosialogangliosides

(43) Date of publication of application: 12.05.1993
(73) Proprietor: Technochemical Corporation S.A., Montevideo (UY)
(72) Inventor: Mammarella, Carlos, Buenos Aires (AR); Cumar, Federico A., Cordoba (AR); Rodriguez, Pablo, Cordoba (AR)
(74) Representative: Pearce, Anthony Richmond

(56) References cited:
- EP-A- 0 004 214
- EP-A- 0 451 267
- FR-A- 2 585 707
- CHEMICAL ABSTRACTS, vol. 87, no. 15, 10 October 1977, Columbus, OH (US); A.L. TERENCE et al., p. 274, no. 113888/

## Description

This invention relates to a method of obtaining monosialoganglioside -G_{M1}- (a glycosphingolipid that has a sialic acid (N-acetylneuraminic acid) moiety bound to monosaccharides as head groups, which gives a net charge to the polar head at neutral pH, and also has a non-polar carbohydrate moiety) from products containing a mixture of gangliosides.

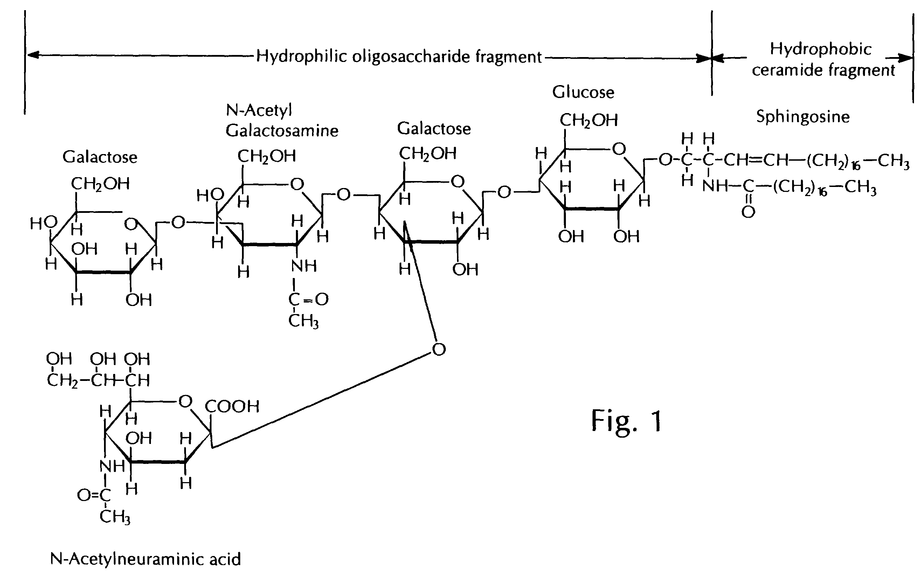

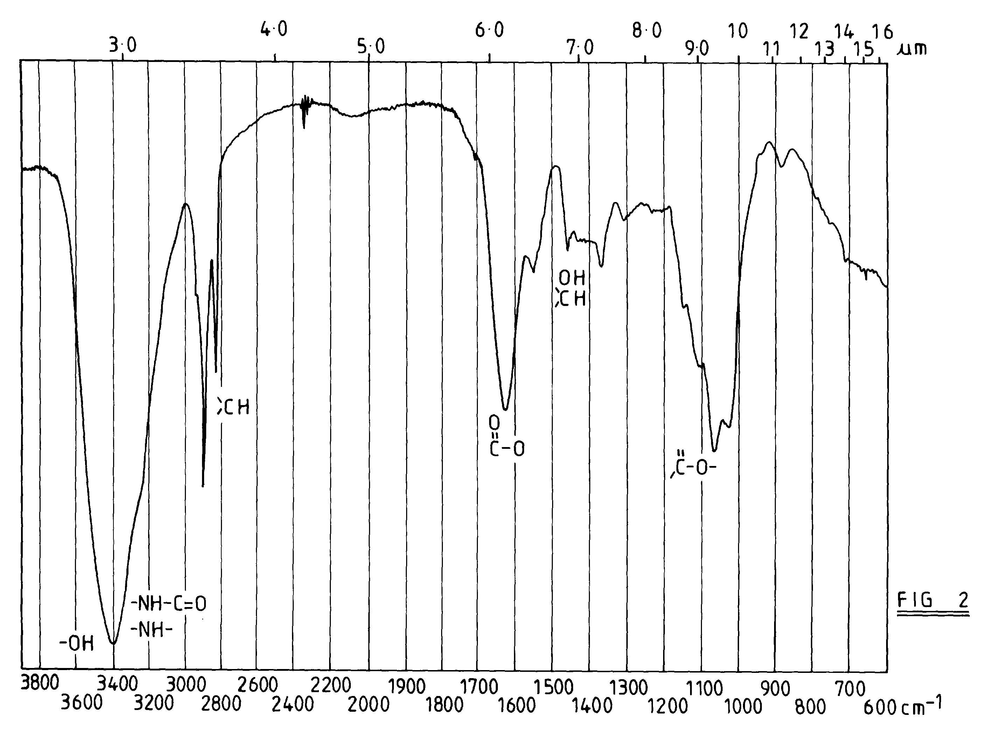

Glycosphingolipids are found in large quantities together with other lipids (e.g. phospholipids) in mammalian brains, mainly in the cortex. In bovine mammals, the content of glycosphingolipids is high enough for them to be extracted for commercial purpose. In a chemical composition of these compounds, units of monosaccharides, sialic acid and a non-polar fragment, the ceramide which is composed of a natural amino alcohol, sphingosine, forming an amide bond with fatty acids, are present. The units of monosaccharides are mainly galactose, glucose and an amino hexose, N-acetylgalactosamine. Furthermore, they contain N-acetyl (and/or glycolyl) neuraminic acid (sialic acid). All glycosphingolipids containing such acid belong to the group of acid glycosphingolipids, and they are classified under the specific name of gangliosides. More than sixty different ganglioside molecules containing different structures have been detected. According to the number of units of sialic acid, they are grouped as monosialogangliosides, disialogangliosides, trisialogangliosides and tetrasialogangliosides, which are mentioned for short as G_{M}, G_{D}, G_{T} and G_{Q}, respectively. The disappearance of the last residue of sialic acid produces G_{A1}, an asialosphingolipid of G_{M1}. A numerical suffix, N (equal to 5-n, where n indicates the number of units of neutral hexose) is added. The letters (generally a, b) indicate compounds which exhibit isomerism in relation to the positions of the sialic acid moieties with characteristic properties for those isomers eg, a different Rf in thin layer chromatography. Example: G_{D1b} represents a disialoganglioside where n=4 and where the sialic acid moiety exhibits isomerism with reference to G_{D1a}.
Thus, G_{M1} is a compound formed by one unit of sialic acid and four units of hexose. This carbohydrate fragment is bound to a ceramide. It is composed of sphingosine forming an amide bond with a fatty acid fragment. More than one fatty acid has been identified by HPLC. They are mainly of C₁₈ and C₂₀. (Hiroki NAKABAEYASHI, Masao IWAMORI and Yoshitaka NAGAI), J. Biochem., 96, 977-984 (1984). The structure of G_{M1} is illustrated in attached Fig. 1 and by the following schematic formula:- 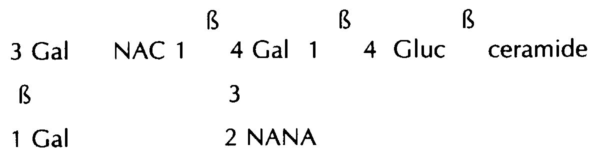 Gal = galactose; Gluc= glucose;
NANA=N-acetylneuraminic acid; Gal Nac= N-acetyl-galactosamine. In the attached Fig.2, the IR spectrum of G_{M1} is shown, in which characteristic peaks corresponding to the C-C, C-OH, C-H, N-C and N-H bonds are indicated.

### CONSTANTS AND CHARACTERISTICS OF THE PRODUCT

Molecular formula: C₇₃H₁₃₁O₃₁N₃ (more abundant component). According to the suggested structure, MW is 1546.80. Melting point: 207°-232°(d).

Appearance: white powder, slightly cream. Solubility: soluble in water. Soluble in mixtures of methanol-water, methanol-chloroform and methanol-chloroform-water. Practically insoluble in methanol, ethanol, acetone, chloroform, ethyl ether and light aliphatic hydrocarbons (petroleum ether). Infra red spectrum:- see the drawing.

### Specifications for finished product (lyophilized).

### a) Identity test

1) Infra red spectrum superimposable to the standard.
2) There must be a coincidence of R_{f} with an authentic sample of it using thin layer chromatography in the different systems of this patent.

### b) Humidity

The lyophilized product must contain not more than 0.5% of water.

### c) Residual solvents

It must contain not more than 0.1%

### d) Melting point

The melting point must not be depressed when the substance is mixed with an authentic sample of it.

### DESCRIPTION OF THE INVENTION

The monosialoganglioside G_{M1} is found in the natural mixture at concentrations of 20% by weight. Therefore, the obtaining of G_{M1} by isolation is not economically attractive. The transformation of the mixture of gangliosides G_{M1} by enzymatic or chemical hydrolysis is the most viable industrial method. In DE-A-3624816, a chemical method for obtaining G_{M1} is described wherein the sialic acid is first separated in units hydrolyzing di- and tri-sialogangliosides present in the natural mixture with dilute inorganic or organic acids; secondly, the portions thus obtained are chromatographically separated and purified in silica gel columns. However, there is available literature about the use of enzymes in this transformation. Both the enzymatic and chemical methods are based on the kinetic control of the reaction. The rate of transformation of G_{T} into C_{D} and C_{D} into G_{M1} is high. In contrast, the hydrolysis rate of G_{M1} into G_{A1} is low. If the experimental conditions are controlled, it is possible to hydrolyze the mixture selectively and obtain G_{M1} in high yield. The enzymatic hydrolysis using neuraminidase has been described in several publications, eg
1) Richard Kuhn et al., Chem. Ber., 96, 866 (1963),
2) Keiko Nohara-Uchida et al., J.Biochem., 103, 840/2 (1988),
3) Myers Metal, Biochem. 23, 1442/8 (1984),
4) Drzernick R. et al., Histochem. Journal, 5, 271/90 (1973),
5) Schauer R. et al., Hoppe-Seyler's Z. Physiol. Chem. 349 961/8 (1968),
6) S. Hakonori et al., Biochem., 8, 5082/88 (1969),
7) Welger D.A. et al., J. Neurochem., 20, 607/12 (1973), and
8) Lipovak V. et al., J. Biol. Chem., 246 (24), 7642 (1971).

EP-A-0451267 discloses a process for preparing G_{M1} from gangliosides using neuraminidase isozyme S derived from bacteria of the genus *Arthrobacter,* wherein an aqueous solution containing the gangliosides and a buffer (pH about 3-6) is incubated with an aqueous solution of the neuraminidase followed by extraction of G_{M1} with a mixture of chloroform and methanol, and lyophilisation.

Hoppe-Seyler's Z. Physiol. Chem, vol 358. no. 7,1977, pages 789-795 discloses the immobilization of neuraminidase derived from *Clostridium perfringens*, the increased stability under various conditions of the immobilized enzymes relative to the soluble enzyme, and the use of the immobilized enzymes to cleave various substrates including G_{D1a} micelles and human erythrocytes.

According to the present invention, there is provided a method of obtaining monosialoganglioside (G_{M1}) from an aqueous solution containing a mixture of gangliosides by incubating the solution in the presence of neuroaminidase so as to hydrolyse di-, tri- and/or tetra-sialogangliosides therein to enrich the G_{M1} content of the mixture, extracting G_{M1} from the reaction solution by a procedure involving the use of a solvent mixture containing an aliphatic alcohol and a haloalkane, and lyophilising, characterised in that the incubating is effected at a pH of from 5 to 5.5 in the presence of an immobilized neuroaminidase obtained from *Clostridium perfringens,* the resultant solution is dried, the residue is re-dissolved in a solvent mixture comprising an aliphatic alcohol, water and a haloalkane, the resultant solution is subjected to exclusion chromatography, the fraction(s) containing the G_{M1} are collected, and such fraction(s) are lyophilized.

In the present application, the hydrolysis of the natural mixture of gangliosides to G_{M1} is effected using an immobilized neuraminidase obtained from *Clostridium perfringens* at a pH of 5 to 5.5. This method allows G_{M1} to be obtained in very high yield and purity. Considering that during the transformation of the gangliosides with more than one unit of sialic acid into monosialo, the free sialic acid is formed, it is necessary to eliminate it as well as the traces of G_{D1} present when the enzymatic reaction is stopped. Under the conditions described in this invention, no G_{A1} is present. This purification is carried out by filtration chromatography through gels (exclusion chromatography), eluting with mixtures of chloroform:methanol: water, more preferably with a volume ratio 60:30:4.5. The passage of the transformation product through Sephadex® G50 (Sephadex® G25 may also be used) allows G_{M1} of high purity to be obtained. Sephadex® G50 is made of dextran crosslinked with epichlorhydrin. The pH range is chosen to obtain a high rate of transformation in a favourable time. The main advantage of this method is that the immobilized enzyme can be generated and used again. In this way, the inconvenience of its high price is overcome and our method is industrially viable.

The enzymatic activity depends on the pH as will be apparent from the following Examples.

### EXAMPLES:

### Example 1: Experiment at pH: 5.

a) Insoluble neuraminidase (Sigma type VI A), of *Clostridium perfringens,* bound to agarose: 5.3 units per gram of gangliosides to be hydrolyzed.
b) Sodium acetate/acetic acid buffer, at pH 5.0, 23.7 ml per gram of gangliosides to be hydrolyzed. Incubation temperature of the mixture: 37°C. Hydrolysis profile:-

| % NANA evolved with reference Hydrolysis time (Hours) to the theoretical amount | |
|---|---|
| 0.5 | 10.81 |
| 1 | 19.00 |
| 2 | 22.30 |
| 3 | 32.63 |
| 4 | 32.61 |
| 19.5 | 42.40 |
| 20 | 43.48 |
| 21 | 44.90 |
| 22 | 52.20 |
| 23 | 53.30 |
| 24 | 53.30 |
| 25 | 54.60 |

### Example 2: Experiment at pH: 5.3

a) Insoluble neuraminidase (Sigma type VI A) of *Clostridium perfringens* bound to agarose: 5.3 units per gram of gangliosides to be hydrolyzed.
b) Total gangliosides (natural mixture).
c) Sodium acetate/acetic acid buffer, at pH 5.3, 23.7 ml per gram of gangliosides. Incubation temperature of the mixture: 37°C. Hydrolysis profile:-

| % NANA evolved with reference Hydrolysis time (Hours) to the theoretical amount | |
|---|---|
| 0.5 | 8.65 |
| 1 | 18.42 |
| 2 | 24.60 |
| 3 | 32.43 |
| 4 | 49.00 |
| 5 | 51.34 |
| 15.5 | 64.00 |
| 16 | 66.50 |
| 23 | 93.00 |
| 24 | 103.12 |
| 25 | 103.70 |

### Example 3: Experiment at pH: 5.5

a) Insoluble neuraminidase (Sigma type VI A) of *Clostridium perfringens* bound to agarose: 5.3 units per gram of gangliosides.
b) Total gangliosides (natural mixture).
c) Sodium acetate/acetic acid buffer, at pH 5.5, 23.7 ml per gram of gangliosides. Incubation temperature: 37°C. The hydrolysis profile is as follows:-

| % NANA evolved with reference Hydrolysis time (Hours) to the theoretical amount | |
|---|---|
| 0.5 | 7.40 |
| 1 | 19.00 |
| 2 | 29.35 |
| 3 | 54.00 |
| 4 | 60.00 |
| 5 | 76.00 |
| 6 | 81.74 |
| 6.5 | 82.00 |
| 24 | 88.00 |
| 25 | 91.30 |
| 26 | 95.70 |
| 27 | 98.00 |
| 28 | 99.00 |
| 28.5 | 99.24 |
| 29 | 102.20 |
| 30 | 103.30 |

The working method with the immobilized enzyme can be batchwise. Once the reaction is finished, we proceed according to the following Example:
Example 4: The enzyme immobilized in agarose is centrifuged, the supernatant containing G_{M1} is separated and is kept till it is purified. The residue is washed twice with the buffer solution at the working pH, and twice with a solution 2.0 M (NH₄)₂.SO₄, at pH 7.0. It is kept in this medium and can be used again more than five times.
Example 5: A solution of G_{M1} obtained according to Examples 1 to 3, from 1 g of total gangliosides (natural mixture), is taken. It is dried in a rotary evaporator at 40-50°C. The residue is taken up in a mixture of chloroform:methanol:water in a respective volume ratio of 60:30:4.5, and the solution obtained is passed through a Sephadex column G50 containing 10 g of Sephadex in the above mentioned mixture of solvents. The fractions are analyzed by thin layer chromatography (J. Neurochem. 10, 613 (1963), J. Chromatog. 106, 425 (1975) revealing either with resorcinol reagent (Biochem. Biophys. Acta, 24, 604 (1957) or with iodine, and those containing pure G_{M1} are put together. The assembled fractions are lyophilized.

## Claims

1. A method of obtaining monosialoganglioside (G_{M1}) from an aqueous solution containing a mixture of gangliosides by incubating the solution in the presence of neuroaminidase so as to hydrolyse di-, tri- and/or tetra-sialogangliosides therein to enrich the G_{M1} content of the mixture, extracting G_{M1} from the reaction solution by a procedure involving the use of a solvent mixture containing an aliphatic alcohol and a haloalkane, and lyophilising, characterised in that the incubating is effected at a pH of from 5 to 5.5 in the presence of an immobilized neuroaminidase obtained from *Clostridium perfringens,* the resultant solution is dried, the residue is re-dissolved in a solvent mixture comprising an aliphatic alcohol, water and a haloalkane, the resultant solution is subjected to exclusion chromatography, the fraction(s) containing the G_{M1} are collected, and such fraction(s) are lyophilized.

2. A method according to claim 1, characterized in that said exclusion chromatography is effected using a column of dextran crosslinked with epichlorhydrin.

3. A method according to claim 1 or 2, characterized in that said solvent mixture is a mixture of chloroform, methanol and water.

4. A method according to claim 3, characterized in that the chloroform, methanol and water are in a volume ratio 60:30:4.5, respectively.

5. A method according to any preceding claim, characterized by the further steps of washing the immobilized enzyme after the hydrolysis, and reusing the washed immobilized enzyme in the incubating step.

## Patentansprüche

1. Verfahren zur Herstellung von Monosialogangliosid (G_{M1}) aus einer wäßrigen Lösung, die ein Gemisch von Gangliosiden enthält, durch Inkubieren der Lösung in Anwesenheit von Neuraminidase, derart, um darin Di-, Tri- und/oder Tetrasialoganglioside zu hydrolysieren. um den G_{M1}-Gehalt des Gemisches anzureichern, Extrahieren des G_{M1} aus der Reaktionslösung mittels eines Verfahrens, das die Verwendung eines Lösungsmittelgemisches einschließt, das einen aliphatischen Alkohol und ein Halogenalkan enthält. und Gefriertrocknen, dadurch gekennzeichnet, daß das Inkubieren bei einem pH von 5 bis 5,5 in Anwesenheit einer immobilisierten Neuraminidase. erhalten aus *Clostridium perfringens.* ausgeführt wird, die entstehende Lösung getrocknet wird, der Rückstand von neuem in einem Lösungsmittelgemisch aufgelöst wird, das einen aliphatischen Alkohol, Wasser und ein Halogenalkan umfaßt, die entstehende Lösung der Ausschlußchromatographie unterworfen wird, die das G_{M1} enthaltende(n) Fraktion(en) gesammelt wird (werden) und die derartige Fraktion(en) gefriergetrocknet wird (werden).

2. Verfahren nach Anspruch 1, dadurch gekennzeichnet, daß die Ausschlußchromatographie unter Verwendung einer Säule mit durch Epichlorhydrin vernetztem Dextran ausgeführt wird.

3. Verfahren nach Anspruch 1 oder 2. dadurch gekennzeichnet, daß das Lösungsmittelgemisch ein Gemisch aus Chloroform, Methanol und Wasser ist.

4. Verfahren nach Anspruch 3. dadurch gekennzeichnet, daß das Chloroform, Methanol und Wasser in einem Volumenverhältnis in dieser Reihenfolge von beziehungsweise 60:30:4,5 vorliegen.

5. Verfahren nach einem der vorhergehenden Ansprüche. gekennzeichnet durch die weiteren Schritte des Waschens des immobilisierten Enzyms nach der Hydrolyse und Wiederverwendung des gewaschenen immobilisierten Enzyms in dem Inkubationsschritt.

## Revendications

1. Procédé de préparation du monosialoganglioside (G_{M1}) à partir d'une solution aqueuse contenant un mélange de gangliosides, en incubant la solution en présence de neuroaminidase de façon à hydrolyser les di-. tri- et/ou tétra-sialogangliosides qui s'y trouvent et enrichir la teneur en G_{M1} du mélange. en extrayant le G_{M1} de la solution réactionnelle par un mode opératoire comprenant l'utilisation d'un mélange de solvants contenant un alcool aliphatique et un halogénoalcane, et en lyophilisant, caractérisé en ce que l'incubation est effectuée à un pH de 5 à 5.5 en présence d'une neuroaminidase immobilisée obtenue à partir de *Clostridium perfringens,* la solution résultante est séchée, le résidu est redissous dans un mélange de solvants comprenant un alcool aliphatique, de l'eau et un halogénoalcane, la solution résultante est soumise à une chromatographie d'exclusion. la (les) fraction(s) contenant le G_{M1} est (sont) recueillie(s) et une (de) telle(s) fraction(s) est (sont) lyophilisée(s).

2. Procédé selon la revendication 1, caractérisé en ce que ladite chromatographie d'exclusion est effectuée en utilisant une colonne de dextrane réticulé avec de l'épichlorhydrine.

3. Procédé selon la revendication 1 ou 2, caractérisé en ce que ledit mélange de solvants est un mélange de chloroforme, de méthanol et d'eau.

4. Procédé selon la revendication 3, caractérisé en ce que le chloroforme, le méthanol et l'eau sont dans un rapport volumique de 60:30:4,5. respectivement.

5. Procédé selon l'une quelconque des revendications précédentes. caractérisé par les étapes supplémentaires de lavage de l'enzyme immobilisée après l'hydrolyse et de réutilisation de l'enzyme immobilisée lavée dans l'étape d'incubation.
